# EUROPEAN PATENT SPECIFICATION

(11) **EP 2 995 243 B1**
(45) Date of publication and mention of the grant of the patent: **17.02.2021**
(21) Application number: 15180223.8
(22) Date of filing: 07.08.2015
(51) Int. Cl.: A61B 5/00, A61B 5/024

(54) **WRIST-WORN APPARATUS FOR OPTICAL HEART RATE MEASUREMENT**
AM HANDGELENK GETRAGENE VORRICHTUNG ZUR OPTISCHE HERZFREQUENZMESSUNG
APPAREIL PORTÉ AU POIGNET QUI MESURE LE RYTHME CARDIAQUE

(30) Priority: 09.09.2014 US 201414481448
(43) Date of publication of application: 16.03.2016
(73) Proprietor: Polar Electro Oy, 90440 Kempele (FI)
(72) Inventor: Kokkoneva, Olli, 90440 Kempele (FI); Lumme, Lauri, 90440 Kempele (FI)
(74) Representative: Kolster Oy Ab

(56) References cited:
- WO-A1-2013/132844
- BE-A- 356 967
- CN-A- 101 138 491
- US-A1- 2005 257 581
- US-A1- 2009 168 612
- US-A1- 2010 302 914
- US-A1- 2014 206 954
- US-A1- 2015 046 095

## Description

### FIELD OF THE INVENTION

The invention relates generally to optical heart activity measurement. More particularly, the present invention relates to using a wrist-worn apparatus to measure heart activity.

### BACKGROUND OF THE INVENTION

Heart activity measurement has become more and more popular way to measure user's activity level during everyday activities and training. Patent application publication US 2009/0168612 A1 and WO 2013/132844 A1 discloses a wrist-worn apparatus for optical heart rate measurement. Patent application publication US 2010/0302914 A1 discloses a locking system for wristwatch straps and bands. Thus, a demand for solutions making the measurement easier for the user has risen.

### BRIEF DESCRIPTION OF THE INVENTION

According to an aspect, there is provided the subject matter of the independent claim.

According to an aspect, there is provided a wrist-worn apparatus for measuring a user's heart activity, the apparatus comprising: an optical heart activity sensor configured to measure the user's heart activity by optical heart rate measurement; and a wrist strap adapted to physically couple the optical heart rate activity sensor against the user's skin by attaching the wrist-worn apparatus around the user's wrist, the wrist strap comprising a first and a second curved connection members, wherein the first connection member is adapted to slide inside the second connection member, and wherein the wrist strap comprises at least one groove and a fixing claw acting as a counterpart for the at least one groove, wherein the fixing claw is arranged to engage the at least one groove when the first connection member is slid inside the second connection member.

In an embodiment, the wrist strap further comprises a release mechanism to enable disengaging of the fixing claw from the at least one groove.

In an embodiment, the fixing claw and the release mechanism are mechanically coupled together with at least one fixing point.

Some further embodiments are defined in the dependent claims.

### BRIEF DESCRIPTION OF THE DRAWINGS

Embodiments of the present invention are described below, by way of example only, with reference to the accompanying drawings, in which
Figure 1 illustrates an exemplary system for measuring and displaying physical activity-related data in order to monitor a physical activity performed by a user;
Figure 2A illustrates a connection mechanism according to an embodiment of the invention;
Figure 2B illustrates an embodiment of the invention;
Figure 3 illustrates a connection between a fixing claw and at least one groove according to an embodiment of the invention;
Figure 4 illustrates the relation between wrist strap loop tightness and the signal quality;
Figure 5 illustrates an embodiment of the invention;
Figure 6 illustrates a block diagram of a wrist-worn apparatus according to an embodiment of the invention;
Figure 7 illustrates pulse detection according to an embodiment of the invention; and
Figure 8 illustrates an optical heart activity sensor according to an embodiment of the invention.

### DETAILED DESCRIPTION OF THE INVENTION

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

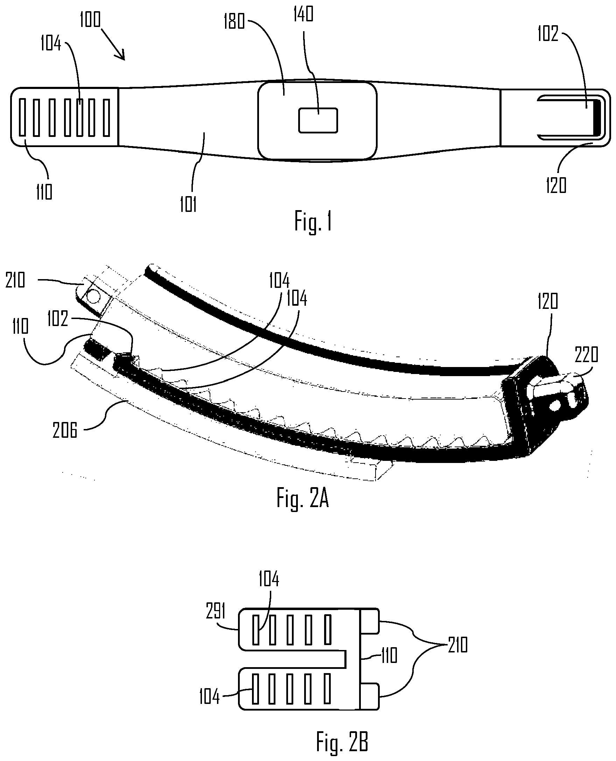

Figure 1 illustrates a wrist-worn apparatus 100 according to an embodiment of the invention. The wrist-worn apparatus 100 may be configured to measure user's physical activity. The wrist-worn apparatus may be a training computer, for example, and thus can be used to measure user's training with sensors of different sort and provide feedback to a user of the wrist-worn apparatus 100. Referring to Figure 1, the wrist-worn apparatus 100, used for measuring a user's heart activity, comprises an optical heart activity sensor 140 configured to measure the user's heart activity by optical heart rate measurement, and a wrist strap 101 adapted to physically couple the optical heart rate activity sensor 140 against the user's skin by attaching the wrist-worn apparatus 100 around the user's wrist, the wrist strap 101 comprising a first curved connection member 110 and a second curved connection member 120, wherein the first connection member 110 is adapted to slide inside the second connection member 120, and wherein the wrist strap 101 comprises at least one groove 104 and a fixing claw 102 acting as a counterpart for the at least one groove 104, wherein the fixing claw 102 is arranged to engage the at least one groove 104 when the first connection member 110 is slid inside the second connection member 120, thus fastening the first and the second connection members 110, 120 together by preventing the enlargement of a loop formed by the wrist strap 101 and allowing the tightening of the loop.

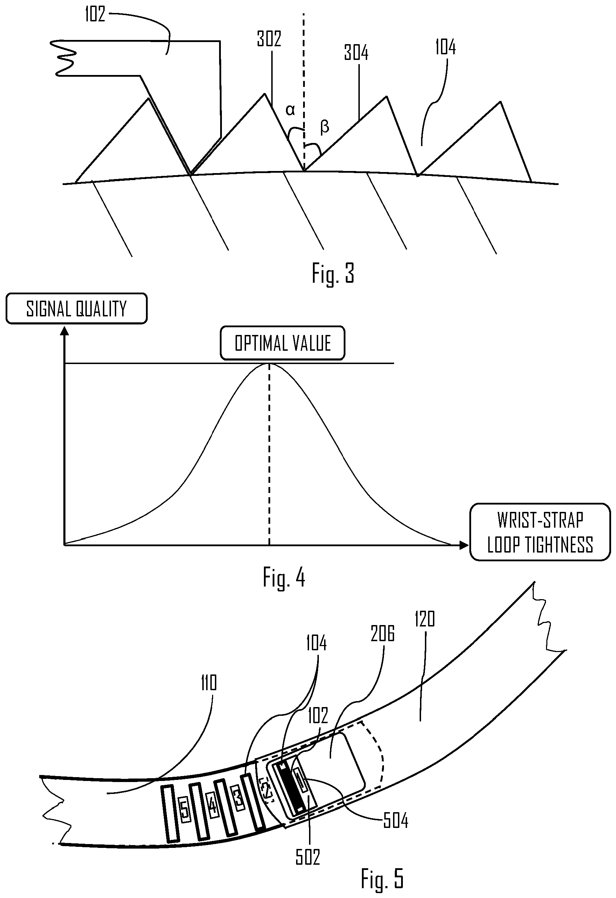

When tightening the loop, formed by the wrist strap 101, the fixing claw 102 may disengage from the at least one groove 104 so that the fixing claw 102 may engage the next groove. Such automatic disengaging may be prevented to the loosening direction by the structure of the fixing claw 102 and the at least one groove 104, which is illustrated later on Figure 3, for example. The described connection mechanism may provide a fast and reliable connection mechanism for the user.

The wrist-worn apparatus 100 may further comprise a main frame 180 which may comprise the above described optical heart activity sensor 140. Figure 1 can be seen as an illustration of the wrist-worn apparatus 100 from the backside, and thus the face shown in Figure 1 may be set against the user's skin. Furthermore, the main frame may comprise a display panel, which may, for example, be located on the front side of the wrist-worn apparatus 100. The described fixing of the wrist strap 101 as a loop around user's wrist may help to achieve an optimal tightness for the optical heart rate sensor 140 to work correctly.

In an embodiment, the first curved connection member 110 is made from thermoplastic material.

In an embodiment, the second curved connection member 120 is made from thermoplastic material.

In an embodiment, the first and/or second connection members 110, 120 are arched.

In an embodiment, the first and/or second connection members 110, 120 are made from metal.

In an embodiment, the wrist-worn apparatus 100 is a wrist-worn training computer.

In an embodiment, the wrist-worn apparatus 100 is a wrist-worn physical activity measurement apparatus.

In an embodiment, the wrist strap 101 is made at least partly from rubber compound. The rubber compound may provide a flexible and comfortable wrist strap 101. The wrist strap 101 may comprise at least one thinning, wherein the at least one thinning may enhance the flexible properties of the wrist strap 101.

In an embodiment, the at least one thinning is formed on the side of the wrist strap 101 which is adapted to be placed against the user's skin.

In an embodiment, the first connection member 110 or the second connection member 120 may comprise the heart activity sensor 140.

In an embodiment, the first connection member 110 or the second connection member 120 comprise at least one groove 104, the wrist strap 101 further comprising a fixing claw 102 acting as a counterpart for the at least one groove 104.

In an embodiment, at least one of the first connection member 110 and the second connection member 120 comprises the at least one groove 104.

In an embodiment, the wrist strap 101 and the flexible claw 102 are of one integral entity. This may mean that the flexible claw 102 is formed, for example, at one end of the wrist strap 101, wherein the said end may be the first or the second connection member 110, 120.

In an embodiment, the fixing claw 102 comprises a flexible element providing the fixing claw 102 flexibility. The flexible element may be an elongated arm, for example. The elasticity may come from the material used to produce the flexible element and/or from the length of the element.

In an embodiment, the fixing claw 102 is a flexible fixing claw.

In an embodiment, the fixing claw 102 is made from thermoplastic material.

In an embodiment, the fixing claw 102 is made from metal.

Still referring to Figure 1, as mentioned above the wrist strap 101 may comprise the first connection member 110 and the second connection member 120. The wrist strap 101 may be of one integral part, wherein the first connection member 110 and the second connection member 120 may be the first and the second ends of the wrist strap 101 respectfully. The wrist strap 101 may comprise a connection member for the main frame 180, wherein the connection member may be used to physically connect the main frame to the wrist strap 101. The connection member may comprise a embedding in which the main frame 180 may be fitted in. Other connection methods may be equally possible, such as gluing.

In an embodiment, the main frame 180 is fixed detachably to the wrist strap 101 in order to allow washing and/or cleaning of the wrist strap 101.

Let us now look closer to the connection mechanism of the wrist strap 101 of Figure 1. As mentioned above, the wrist strap 101 may form a loop when the first and the second connection members 110, 120 are connected together. Figure 2A illustrates the connection mechanism according to an embodiment of the invention. The first connection member 110 may be slid inside the second connection member 120, wherein the fixing claw 102 may fix the connection between the first and second connection member 110, 120 by engaging the at least one groove 104 and preventing the first connection member 110 from sliding to the opposite direction of sliding in to the second connection member 120. The wrist strap 101 may comprise a release mechanism 206 to enable the enlargement of the loop formed by the wrist strap 101 by disengaging the fixing claw 102 from the at least one groove 104. The release mechanism 206 may be physically connected to the fixing claw 102. The release mechanism 206 may work with mechanical force, for example. The release mechanism 206 may be pulled or pressed to disengage or release the fixing claw 102 from the at least one groove 104, thus enabling the loop to be loosened and/or the first connection member 110 to be extracted from the second connection member 120.

Still referring to Figure 2A, the first connection member 110 may comprise a first connector 210 to be used to mechanically connect the first connection member 110 to the wrist strap 101. The first connector 210 may, for example, comprise at least one protrusion with a hole. The at least one protrusion may be fitted to a counterpart in the wrist strap and fixed with a pivot penetrating the hole and at least one part of the wrist strap. The wrist strap 101 may comprise a hole for the said pivot, wherein the hole in the wrist strap 101 is in line with the hole in the at least one protrusion allowing the said pivot to fix the at least one protrusion to the wrist strap 101. The second connection member 120 may comprise a second connector 220 similar to the first connector 210, thus allowing similar connection to the wrist strap as described above.

In an embodiment, the release mechanism 206 comprises a surface, and wherein the surface is arranged to be reaching over at least one edge area of the wrist strap 101. This may help the user to easily use the release mechanism 206.

In an embodiment, the fixing claw 102 is arranged to disengage by applying mechanical force to the surface comprised in the release mechanism 206.

In an embodiment, the fixing claw 102 and the release mechanism 206 are mechanically coupled together with at least one fixing point.

In an embodiment, the fixing claw 102 and the release mechanism 206 are of one integral entity.

In an embodiment, the release mechanism 206 comprises a decoration. The decoration may be a carving or a figure made with bas-relief technique, for example.

In an embodiment, the release mechanism 206 is made from metal.

Figure 2B illustrates an embodiment of the invention. Referring to Figure 2B, the first connection member 110 may comprise one or more protrusions 291. The one or more protrusions 291 may comprise the at least one groove 104. The at least one groove 104 may be comprised only in some of the protrusions 291. Similarly, the fixing claw 102 may comprise one or more engaging members which may engage the at least one groove 104. In an embodiment, the wrist strap 101 comprises a number of fixing claws, similar to fixing claw 102 of Figure 2A, or the fixing claw 102 comprises a number of engaging members, wherein the said numbers are equal to the number of protrusions 291. This may allow the grooves in each of the protrusions 291 to be engaged when the wrist strap 101 is connected.

Figure 3 illustrates the connection between the fixing claw 102 and the at least one groove 104 according to an embodiment of the invention. The at least one groove 104 may comprise a fastening wall 302, a tightening wall 304 and a cavity between the said walls 302, 304. Angle α between a line perpendicular to the curved connection member, such as first or second connection members 110, 120 of Figure 1, and the fastening wall 302 may be smaller than angle β between the said perpendicular line and the tightening wall 304. The tightening wall 304 may allow the first connection member 110 to slide inside the second connection member 120, thus tightening the loop formed by the wrist strap 101 by allowing the fixing claw 102 to disengage automatically when the loop is tightened, and wherein the fastening wall 302 may prevent the fixing claw 102 from disengaging from the at least one groove 104 to the direction opposite to the tightening. The smaller angle α compared to the angle β may mean that the fastening wall 302 is steeper compared to the tightening wall 304. This feature of the at least one groove 104 may provide more mechanical force to the fixing claw 102 when the loop is tried to loosen. Similarly, the same feature may allow the loop to be tightened as the gentler wall may allow the fixing claw 102 to move from one groove to another.

The optical heart rate sensor, such as the optical heart rate sensor 140 of Figure 1, may require firm connection with the user's skin to operate correctly. Figure 4 illustrates the relation between the wrist strap 101 loop tightness and the signal quality. Referring to Figure 4, the signal quality of the optical heart rate measurement may enhance as the loop is tightened. This may happen as the optical heart rate sensor's physical connection may get more stable and firmer. The signal quality may have an optimal value corresponding to certain loop tightness. It needs to be noted that the optimal heart rate measurement may work although the signal quality would not be optimal. As the loop tightness may continue to increase, the signal quality may start to decrease after the optimal value of the signal quality. The signal quality may be between acceptable limits even though the signal quality would not be optimal.

Figure 5 illustrates an embodiment of the invention. The wrist strap of Figure 1 may further comprise a position indicator which may indicate the tightness of the loop formed by the wrist strap. In Figure 5 the position indicator is achieved by making a display hole 502 in the second connection member 120, near to the point where the fixing claw 102 may engage the at least one groove 104. An indicator 504, such as color code, number, characters or other visual markers, may correspond to each individual groove, thus indicating the tightness of the loop. The first connection member 110 and/or the second connection member 120 may comprise the indicators 504. The indicators 504 may be visible at least partially through the hole in the second connection member.

In an embodiment, the position indicator comprises visual indicators 504, wherein at least one of the visual indicators 504 is at least partially visible, when the first connection member 110 is slid inside the second connection member 120. The display hole 502 may not be needed, as the visual indicators 504 may be visible from the side of the wrist strap 101, for example. It may also be possible to make the wrist strap 101 from translucent or transparent materials. This may allow the visual indicator indicating the tightness of the loop to be seen through the second connection member 120, if the visual indicators 504 are comprised in the first connection member 110, for example.

In an embodiment, the first connection member 110 comprises at least part of the position indicator.

In an embodiment, the second connection member 120 comprises at least part of the position indicator.

In an embodiment, the position indication is achieved by making visual indicators to the at least one groove 104 or in vicinity of the at least one groove 104. Thus, the position may be indicated by the visual indicator that is closest to the second connection member 120, but not inside the second connection member 120. It may be possible that the fixing claw 102 is engaging a different groove than the one which is indicated by the said visual indicator.

In an embodiment, the wrist strap 101 further comprises a limiter adapted to limit the loop, formed by the wrist strap 101, from being tightened over a predetermined level. The limiter may be a prolongation for the first connection member 110, which may limit the first connection member 110 from being slid inside second connection member 120 over a predetermined point, as the prolongation may physically touch the base of the second connection member 120. Similarly, the second connection member 120 may have an additional removable piece which may be installed to the base of the second connection member 120. It may also be possible to install a counter piece around the first connection member 110, which may stop the first connection member 110 from being slid inside the second connection member 120 over a predetermined level.

In an embodiment, the wrist strap 101 comprises a locking member, wherein the locking member is adapted to prevent the accidental disengaging of the fixing claw 102. For example, when performing an exercise it may be possible to accidentally produce enough mechanical energy to disengage the fixing claw 102 from the at least one groove 104. The locking member may be a catch or a strap, for example. The catch may be attached to the wrist strap 101 with a hinge-like member that may be able to lock the fixing claw 102 to its engaging position. Similarly, the strap, when connected, may prevent the fixing claw 102 to be disengaged. The locking member may be unlocked to, by the user, to enable tightening or loosening the loop. In an embodiment, the locking member prevents the release mechanism 206 from disengaging the fixing claw 102. When unlocked, the locking member may allow the release mechanism 206 to disengage the fixing claw 102.

In an embodiment, the release mechanism 206 comprises the locking member.

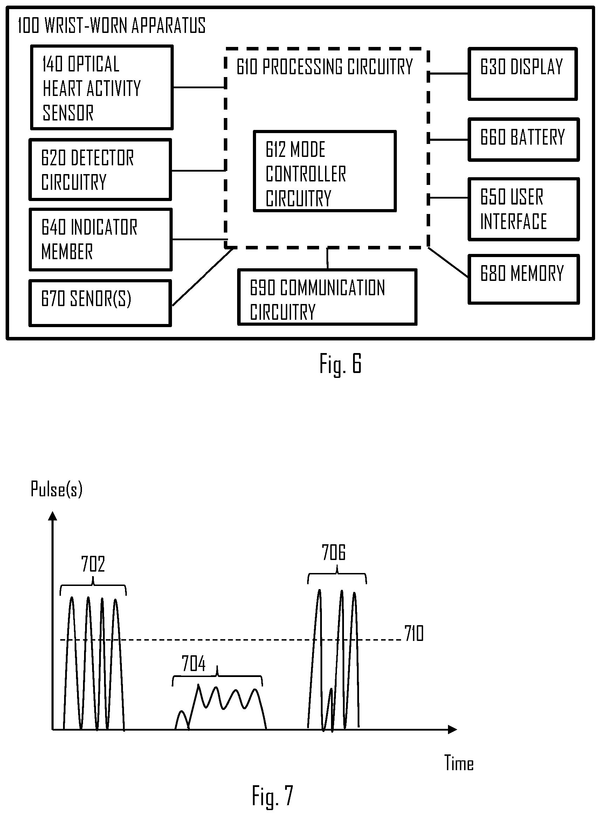

Figure 6 illustrates a block diagram of the wrist-worn apparatus 100 according to an embodiment. The wrist-worn apparatus 100 may comprise a processing circuitry 610, wherein the processing circuitry 610 may be configured to receive and process heart activity related data from the optical heart activity sensor 140. The wrist-worn apparatus 100 may further comprise a detector circuitry 620 for detecting the number of grooves engaged by the fixing claw 102, and wherein the processing circuitry 610 is further configured to receive detected data from the detector circuitry 620 and to calculate the number of grooves engaged by the fixing claw 102 based on the said detected data.

In an embodiment, the detector circuitry 620 is configured to detect vibration produced by the fixing claw 102 engaging the at least one groove 104, and wherein the processing circuitry 610 may further be configured to receive detected vibration data from the detector circuitry 620 and to calculate the number of grooves engaged by the fixing claw 102 based on the said detected vibration data.

In an embodiment, the detector circuitry 620 is configured to detect sound caused by the fixing claw 102 engaging the at least one groove 104.

In an embodiment, the detector circuitry 620 comprises an acceleration sensor. The acceleration sensor may produce acceleration data for the processing circuitry 610. The processing circuitry 610 may deduce, based on the acceleration data, tightness of the loop formed by the wrist strap 101.

Still referring to Figure 6, the wrist-worn apparatus 100 may further comprise a display 630, wherein the number of grooves engaged by the fixing claw 102 may be configured to be displayed on the display 630 with a visual indicator. The visual indicator may, for example, be number, color or combination of the two. Furthermore, the wrist-worn apparatus may comprise an indicator member 640 configured to indicate to the user when the number of grooves engaged by the fixing claw 102 correspond to a predetermined number. The predetermined number may mean a number of grooves engaged by the flexible claw 102, which may be optimal for the signal quality. The predetermined number may also be a value range defining lower and upper limits for the loops tightness. The optimal value or value range may be determined from user history data, for example. Another example is the use of tutorial mode explained later. The history data may comprise the used tightness of the loop in the past.

In an embodiment, the indicator member 640 comprises a vibration member configured to vibrate when the number of grooves engaged by the fixing claw 102 corresponds to a predetermined number. The vibration may help the user to easily and rapidly know whether the wrist strap 101 is tightened correctly.

In an embodiment, the indicator member 640 comprises a speaker or an actuator configured to produce sound when the number of grooves engaged by the fixing claw 102 corresponds to a predetermined number.

Still referring to Figure 6, the processing circuitry 610 may comprise a mode controller circuitry 612. The mode controller circuitry 612 may control different modes of the wrist-worn apparatus 100. The wrist-worn apparatus 100 may comprise at least two different modes: sleep mode and operational mode, wherein entering the sleep mode may cause at least some of the following: dimming of the display 630, turning off the display 630 and deactivation of the optical heart rate sensor 140, and wherein entering the operational mode may cause at least some of the following: undimming of the display 630, turning on the display 630 and activation of the optical heart rate sensor 140. The sleep mode may help to save battery 680 providing the wrist-worn apparatus 100 operational voltage.

The operation mode may be understood as the mode which is used when the wrist-worn apparatus 100 is used by the user. This may mean that the operational mode is always on when the wrist-worn apparatus 100 is connected around the user's wrist. Before entering the operational mode, the processing circuitry 610 may perform a test measurement using the optical heart activity sensor 140. By this test measurement, the processing circuitry 610 may ensure that the wrist-worn apparatus 100 is properly connected around the user's wrist. The test measurement may be performed at a certain time interval when the wrist-worn apparatus 100 is in operational mode. Thus, the processing circuitry 610 may determine is the operational mode still needed. Naturally, if the user is using the wrist-worn apparatus 100 in operational mode and uses the wrist-worn apparatus 100 to measure heart activity, the test measurement may not be needed. It may be understood that the entering to the operational mode may be triggered by some event and the heart activity measurement may be used to ensure that the operational mode should be entered.

In an embodiment, the wrist-worn apparatus 100 is configured to enter operational mode when the detector circuitry 620 detects a predetermined number of consecutive grooves engaged by the fixing claw 102.

In an embodiment, the wrist-worn apparatus 100 is configured to enter operational mode when the detector circuitry 620 detects a predetermined number of consecutive vibrations caused by the fixing claw 102 engaging the at least one groove 104. The described testing measurement may be used to ensure that the wrist-worn apparatus 100 is in fact in use.

The sleep mode may be understood as means for saving energy, and thus battery of the wrist-worn apparatus 100. The entering to the sleep mode may be triggered by some event, such as the opening of the loop formed by the wrist strap 101. The processing circuitry 610 may perform test measurement after an event, such as the opening of the loop, happens. The test measurement may ensure that the wrist-worn apparatus 100 is in fact not in use. There may be situations where the wrist-worn apparatus 100 is used when it is not around the user's wrist. To prevent entering the sleep mode in such cases, the mode controller 612 may receive information from display 630 and/or sensor(s) 670 that indicates that the wrist-worn apparatus 100 is in use.

In an embodiment, the sleep mode is activated when the wrist-worn apparatus 100 is stationary for or over a predetermined time. The time may be, for example, ten seconds. The described testing measurement may be used to ensure that the wrist-worn apparatus 100 is not in fact in use.

In an embodiment, the predetermined time is one minute.

In an embodiment, the predetermined time is five minutes.

In an embodiment, the predetermined time may be adjusted by the user of the wrist-worn apparatus 100.

In an embodiment, the described testing measurements last for ten seconds.

In an embodiment, the described testing measurements last for one minute.

In an embodiment, the wrist-worn apparatus 100 may further comprise a tutorial mode, wherein the mode controller circuitry 612 may be configured to: cause the display 630 to show instructions for the user to tighten or loosen the loop formed by the wrist strap 101 and determine after each displayed instruction, based on the processed heart activity related data, if the quality of the optical heart rate measurement performed by the optical heart activity sensor 140 is between predetermined values. In tutorial mode the wrist-worn apparatus 100 may find the optimal value for tightening the wrist strap 101, as illustrated in Figure 4. The wrist-worn apparatus 100 may be required to be in the operational mode before entering the tutorial mode.

In an embodiment, the tutorial mode is configured to find lower and higher limits for the tightness of the loop, wherein the optical heart activity sensor 140 can measure the user's heart activity with acceptable accuracy. The acceptable accuracy may mean signal quality values, which can be used to determine user's pulse, for example.

In an embodiment, the wrist strap 101 comprises a position detector circuitry configured to detect the position of the first connection member 110 in relation to second connection member 120. The detection may be done also vice versa. The position detector may provide position information for the processing circuitry 610, wherein the processing circuitry 610 may use the information to determine the tightness of the loop formed by the wrist strap 101.

In an embodiment, the wrist-worn apparatus 100 comprises at least one of the following: a temperature sensor, a pressure sensor, a motion sensor and a piezoelectric sensor. Sensor(s) block 670 may comprise the said sensor(s).

In an embodiment, the wrist-worn apparatus 100 comprises a communication circuitry 690. The communication circuitry 690 may be configured to transmit and/or receive data from external devices, such as computers, tablets and mobile phones, for example. The communication circuitry 690 may apply at least one of the following short range device-to-device communication technologies: Near Field Communication (NFC), Radio Frequency Identification (RFID), Bluetooth, Bluetooth Low Energy, wireless local area network (WLAN), ANT or ANT+ by Dynastream, or IEEE 802.15.4. Other short-range device-to-device or network communication protocols are equally possible. The wrist-worn apparatus 100 may utilize NFC for transmitting and receiving data from external devices. NFC circuitry may be also utilized to perform a handshake between the wrist-worn apparatus 100 and another external device, and other communication technologies may be used to transmit or receive the actual data to be transferred. The NFC is a technique enabling radio frequency identification in very short distances.

In an embodiment, the wrist-worn apparatus comprises a user interface 650 configured to allow user to interact with the wrist-worn apparatus 100. The user interface 650 may comprise a touch-screen display formed together with the display 630. Physical buttons for the user to interact with the apparatus are equally possible.

In an embodiment, the wrist-worn apparatus 100 may be connected to a second apparatus, such as tablet, mobile phone or computer, with the communication circuitry 690. The communication circuitry 690 may enable the use of the second apparatus to control the wrist-worn apparatus 100. It may also be possible to load configuration information or software updates to the wrist-worn apparatus 100 using the second apparatus. The configuration information may comprise, for example, algorithms to be used in the detection of wrist strap's 101 loop tightness.

In an embodiment, the communication circuitry 690 is configured to transmit heart activity related data to the second apparatus, wherein the said data causes the second apparatus to display the said data on the display of the second apparatus. This may help the user to review and/or edit the data, as the display may be bigger than the display 630.

Figure 7 illustrates the detection of pulse(s) according to an embodiment of the invention. As mentioned above, the processing circuitry 610 may determine the tightness of the loop, for example, from the vibration pulses caused by the fixing claw 102 engaging the at least one groove 104. Referring to Figure 7, there is shown three different pulse patterns 702-706. The pulse patterns 702-706 need to be understood as exemplary patterns. The patterns observed may comprise any combination or variation of the patterns 702-706 introduced.

The detector circuitry 620 may detect pulse(s) in the pulse patterns 702-706, and the processing circuitry 610 may calculate the number of pulses based on the data received from the detector circuitry 620. In an embodiment, the calculating is done by the detector circuitry 620. The processing circuitry 610 may measure the time which an individual pulse or a pulse pattern lasts and how powerful the pulse is. The processing circuitry 610 may further deduce different things from the pulse patterns 702-706 based on at least some of the measured variables. For example, the processing circuitry 610 may determine that the pulses of the pulse pattern 702 are steep over a predetermined level. The steepness may be determined based on the pulse strength in relation to time. Both upward and downward steepness may be used for the determination. Based on the steepness of the pulse pattern's 702 pulses, the processing circuitry 610 may determine that the pulse pattern 702 is caused by the engaging fixing claw 102. Thus, it may be possible to further determine that the wrist strap 101 has been tightened. More precisely, the wrist strap 101 may have been tightened for the amount of four grooves.

Still referring to Figure 7, the pulse pattern 704 may comprise pulses that are not as steep and/or powerful as the pulse pattern 702. The processing circuitry 610 may determine from the pulse pattern 704 that the steepness and strength of the pulse(s) indicate that the wrist strap 101 is being loosened. The pulse pattern 704 may comprise a first part which indicates that the fixing claw 102 has been disengaged from the at least one groove 104, and a second part which indicates the loosening of the loop. The processing circuitry 610 may determine the amount the loop has been loosened from the time that the second part, indicating the loosening, of the pulse pattern 704 lasts.

The processing circuitry 610 may have pulse strength limit 710 for calculating the pulse(s) caused by the engaging fixing claw 102. The processing circuitry 610 may determine from the structure of the pulse pattern 706 that the pattern 706 is caused by a tightening of the wrist strap 101. However, an individual pulse in the pulse pattern 706 may not go over the pulse strength limit 710. Thus, processing circuitry 610 may determine that only three grooves have been engaged, as one of the pulses may have been erroneous. The errors may be caused by, for example, external force, such as a user's finger touching the sensor(s) observing the pulses or the wrist strap 101.

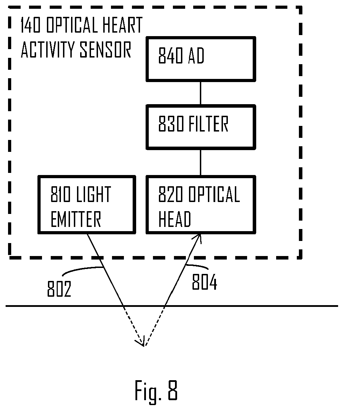

Figure 8 illustrates the optical heart activity sensor 140 according to an embodiment of the invention. The optical heart activity sensor 140 may comprise a light emitter 810 configured to transmit light 802 towards user's skin. The light emitter 810 may comprise a LED, for example. Optical heart activity sensor 140 may further comprise an optical head 820 configured to receive at least some of the bounced light 804 from the user's skin. The light 802, 804 may travel through user's blood veins and/or skin. When the light 802, 804 travels through the veins, it may change and the changes may be observable from the bounced and/or emitted light 804. The changes in the bounced light 804 may indicate different blood fluctuations in the veins. Thus, the pulse or other heart activities may be observable from the detected bounced light 804.

Although the light emitter 810 and the optical head 820 are shown as single units in the Figure 8, it may be possible to use multiple light emitters and optical heads. Optical heart activity sensor 140 may comprise a filter 830 and analog-to-digital (AD) converter 840. These may be configured to filter the signal, detected by the optical head 820 from the bounced light 804, and to convert the analog signal to a digital signal. It needs to be noted that the wrist-worn apparatus 100 may comprise the filter 830 and the AD converter 840. The measured, filtered and converted signal may be transmitted to the processing circuitry 610, as shown in Figure 6. The heart activity information provided by the sensor 140 may be displayed to the user using the display 630.

In an embodiment, the first connection member 110 or the second connection member 110 comprises at least one protrusion, wherein the wrist strap comprises a groove working as a counterpart for the at least one protrusion, and wherein the at least one protrusion may engage the groove, thus connecting the first and second connection members 110, 120 together. The described connection mechanism may use similar solutions that are described above. The at least one protrusion may, for example, produce vibration as it engages the said groove. Similarly, release mechanism 206 may be applicable to the described connection mechanism. The groove may be comprised in the first connection member 110 or the second connection member 120.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The techniques and methods described herein may be implemented by various means. For example, these techniques may be implemented in hardware (one or more devices), firmware (one or more devices), software (one or more modules), or combinations thereof. For a hardware implementation, the apparatus(es) of embodiments may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, described with regard thereto, and they are not limited to the precise configurations set forth in the given figures, as will be appreciated by one skilled in the art.

Embodiments as described may also be carried out in the form of a computer process defined by a computer program. The computer program may be in source code form, object code form, or in some intermediate form, and it may be stored in some sort of carrier, which may be any entity or device capable of carrying the program. For example, the computer program may be stored on a computer program distribution medium readable by a computer or a processor. The computer program medium may be, for example but not limited to, a record medium, computer memory, read-only memory, electrical carrier signal, telecommunications signal, and software distribution package, for example. Coding of software for carrying out the embodiments as shown and described is well within the scope of a person of ordinary skill in the art.

Even though the invention has been described above with reference to an example according to the accompanying drawings, it is clear that the invention is not restricted thereto but can be modified in several ways within the scope of the appended claims. Therefore, all words and expressions should be interpreted broadly and they are intended to illustrate, not to restrict, the embodiment. It will be obvious to a person skilled in the art that, as technology advances, the inventive concept can be implemented in various ways. Further, it is clear to a person skilled in the art that the described embodiments may, but are not required to, be combined with other embodiments in various ways.

## Claims

1. A wrist-worn apparatus (100) for measuring a user's heart activity, the apparatus comprising:
an optical heart activity sensor (140) configured to measure the user's heart activity by optical heart rate measurement;
a wrist strap (101) adapted to physically couple the optical heart rate activity sensor against the user's skin by attaching the wrist-worn apparatus around the user's wrist, the wrist strap comprising a first (110) and a second (120) curved connection members, wherein the first connection member is adapted to slide inside the second connection member, and wherein the wrist strap comprises at least one groove (104) and a fixing claw (102) acting as a counterpart for the at least one groove, wherein the fixing claw is arranged to engage the at least one groove when the first connection member is slid inside the second connection member;
a processing circuitry (610) configured to receive and process heart activity related data from the optical heart activity sensor; and a detector circuitry (620) for detecting the number of grooves engaged by the fixing claw, and wherein the processing circuitry is further configured to receive detected data from the detector circuitry and to calculate the number of grooves engaged by the fixing claw based on the detected data.

2. The wrist-worn apparatus of claim 1, wherein in at least one of the first connection member and the second connection member comprises the at least one groove.

3. The wrist-worn apparatus of any preceding claim, wherein the wrist strap and the flexible claw are of one integral entity.

4. The wrist-worn apparatus of any preceding claim, wherein the at least one groove comprises a fastening wall, a tightening wall and a cavity between the said walls, wherein angle between a line perpendicular to the curved connection member and the fastening wall is smaller than angle between the said line and the tightening wall, wherein the tightening wall allows the first connection member to slide inside the second connection member tightening the loop formed by the wrist strap by allowing the fixing claw to disengage automatically when the loop is tightened, and wherein the fastening wall prevents the fixing claw from disengaging from the at least one groove to the direction opposite to the tightening.

5. The wrist-worn apparatus of any preceding claim, wherein the wrist strap further comprises a release mechanism to enable disengaging of the fixing claw from the at least one groove.

6. The wrist-worn apparatus of any preceding claim, wherein the wrist strap comprises a cavity for the release mechanism.

7. The wrist-worn apparatus of any preceding claim, wherein the release mechanism comprises a surface, and wherein the surface is arranged to reach over at least one edge area of the wrist strap.

8. The wrist-worn apparatus of any preceding claim, wherein the fixing claw is arranged to disengage by applying mechanical force to the surface comprised in the release mechanism.

9. The wrist-worn apparatus of any preceding claim, wherein the wrist strap further comprises a position indicator indicating the tightness of the loop formed by the wrist strap.

10. The wrist-worn apparatus of claim 9, wherein the position indicator comprises visual indicators, wherein at least one of the visual indicators is at least partially visible, when the first connection member is slid inside the second connection member.

11. The wrist-worn apparatus of any preceding claim, wherein the detector circuitry comprises an acceleration sensor.

12. The wrist-worn apparatus of any preceding claim, further comprising:
a display, and wherein the number of grooves engaged by the fixing claw is configured to be displayed on the display with a visual indicator.

13. The wrist-worn apparatus of claim 12, further comprising:
an indicator member configured to indicate to the user when the number of grooves engaged by the fixing claw correspond to a predetermined number.

14. The wrist-worn apparatus of any preceding claim, wherein the wrist-worn apparatus comprises at least two modes: sleep mode and operational mode, wherein entering the sleep mode causes at least some of the following: dimming of the display, turning off the display and deactivation of the optical heart rate sensor, and wherein entering the operational mode causes at least some of the following: undimming of the display, turning on the display and activation of the optical heart rate sensor.

15. The wrist-worn apparatus of claim 14, wherein the wrist-worn apparatus is configured to enter operational mode when the detector circuitry detects a predetermined number of consecutive grooves engaged by the fixing claw.

16. The wrist-worn apparatus of any of claim, wherein the wrist-worn apparatus further comprises a tutorial mode, wherein the processing circuitry is configured to: cause the display to display instructions for the user to tighten or loosen the loop formed by the wrist strap and determine after each displayed instruction, based on the processed heart activity related data, if the quality of the optical heart rate measurement performed by the optical heart activity sensor is between predetermined values.

17. The wrist-worn apparatus of any preceding claim, wherein the wrist strap further comprises a limiter member adapted to limit the loop, formed by the wrist strap, from being tightened over a predetermined level.

## Patentansprüche

1. Am Handgelenk zu tragende Vorrichtung (100) zum Messen der Herzaktivität eines Benutzers, wobei die Vorrichtung umfasst:
einen optischen Herzaktivitätssensor (140), der dafür konfiguriert ist, die Herzaktivität des Benutzers durch optische Messung der Herzfrequenz zu messen;
ein Handgelenkband (101) zum physischen Koppeln des optischen Herzaktivitätssensors mit der Haut des Benutzers durch die Befestigung der am Handgelenk zu tragenden Vorrichtung um das Handgelenk des Benutzers, wobei das Handgelenkband ein erstes (110) und ein zweites (120) gebogenes Verbindungselement umfasst, wobei das erste Verbindungselement dafür ausgelegt ist, in das zweite Verbindungselement zu gleiten, und wobei das Handgelenkband zumindest eine Nut (104) und eine Befestigungsklaue (102) umfasst, die als Gegenstück zu der zumindest einen Nut fungiert, wobei
die Befestigungsklaue derart ausgelegt ist, dass sie in die zumindest eine Nut eingreift, wenn das erste Verbindungselement in das zweite Verbindungselement geschoben wird;
eine Verarbeitungsschaltung (610), die dafür konfiguriert ist, Daten im Zusammenhang mit der Herzaktivität von dem optischen Herzaktivitätssensor zu empfangen und zu verarbeiten; und eine Detektorschaltung (620) zum Erkennen der Anzahl von Nuten, die sich durch die Befestigungsklaue in Eingriff befinden, und wobei die Verarbeitungsschaltung ferner dafür konfiguriert ist, erfasste Daten von der Detektorschaltung zu empfangen und die Anzahl von Nuten, die sich durch die Befestigungsklaue in Eingriff befinden, basierend auf den erfassten Daten zu berechnen.

2. Am Handgelenk zu tragende Vorrichtung nach Anspruch 1, wobei mindestens einer das erste Verbindungselement und das zweite Verbindungselement die mindestens eine Nut umfasst.

3. Am Handgelenk zu tragende Vorrichtung nach einem vorstehenden Ansprüche, wobei das Handgelenkband und die flexible Klaue eine integrale Einheit bilden.

4. Am Handgelenk zu tragende Vorrichtung nach einem der vorstehenden Ansprüche,
wobei die mindestens eine Nut eine Befestigungswand, eine Spannwand und einen Hohlraum zwischen diesen Wänden umfasst, wobei der Winkel zwischen einer Linie senkrecht zu dem gebogenen Verbindungselement und der Befestigungswand kleiner ist als der Winkel zwischen der Linie und der Spannwand, wobei es die Spannwand ermöglicht, dass das erste Verbindungselement in das zweite Verbindungselement gleitet, wodurch die durch das Handgelenkband gebildete Schlaufe gespannt wird, indem es der Befestigungsklaue ermöglicht wird, sich automatisch zu lösen, wenn die Schlaufe gespannt wird, und wobei die Befestigungswand verhindert, dass sich die Befestigungsklaue aus der zumindest einen Nut in die dem Spannen entgegengesetzte Richtung löst.

5. Am Handgelenk zu tragende Vorrichtung nach einem der vorstehenden Ansprüche, wobei das Handgelenkband ferner einen Freigabemechanismus umfasst, der das Lösen der Befestigungsklaue aus der zumindest einen Nut ermöglicht.

6. Am Handgelenk zu tragende Vorrichtung nach einem vorstehenden Ansprüche, wobei das Handgelenkband einen Hohlraum für den Freigabemechanismus umfasst.

7. Am Handgelenk zu tragende Vorrichtung nach einem vorstehenden Ansprüche, wobei der Freigabemechanismus eine Fläche umfasst, und wobei sich die Fläche über mindestens einen Randbereich des Handgelenkbandes hinaus erstreckt.

8. Am Handgelenk zu tragende Vorrichtung nach einem der vorstehenden Ansprüche, wobei die Befestigungsklaue dafür ausgelegt ist, sich durch das Anwenden mechanischer Kraft auf die Fläche, die Teil des Freigabemechanismus ist, zu lösen.

9. Am Handgelenk zu tragende Vorrichtung nach einem der vorstehenden Ansprüche, wobei das Handgelenkband ferner einen Positionsanzeiger umfasst, der die Spannung der von dem Handgelenkband gebildeten Schlaufe anzeigt.

10. Am Handgelenk zu tragende Vorrichtung nach Anspruch 9, wobei der Positionsanzeiger optische Anzeigen umfasst, wobei zumindest eine der optischen Anzeigen zumindest teilweise sichtbar ist, wenn das erste Verbindungselement in das zweite Verbindungselement geschoben wird.

11. Am Handgelenk zu tragende Vorrichtung nach einem der vorstehenden Ansprüche, wobei die Detektorschaltung einen Beschleunigungssensor umfasst.

12. Am Handgelenk zu tragende Vorrichtung nach einem der vorstehenden Ansprüche, die ferner umfasst:
ein Display, und wobei die Anzahl von Nuten, in denen die Befestigungsklaue im Eingriff ist, derart konfiguriert ist, dass sie auf dem Display mit einer optischen Anzeige angezeigt wird.

13. Am Handgelenk zu tragende Vorrichtung nach Anspruch 12, die ferner umfasst:
ein Anzeigeelement, das dafür konfiguriert ist, dem Benutzer anzuzeigen, wenn die Anzahl von Nuten, in denen die Befestigungsklaue im Eingriff ist, einer vorgegebenen Anzahl entspricht.

14. Am Handgelenk zu tragende Vorrichtung nach einem der vorstehenden Ansprüche, wobei die am Handgelenk zu tragende Vorrichtung mindestens zwei Modi umfasst: Schlafmodus und Betriebsmodus, wobei das Wechseln in den Schlafmodus zumindest einige der folgenden Vorgänge bewirkt: Dimmen des Displays, Ausschalten des Displays und Deaktivieren des optischen Herzfrequenzsensors, und wobei das Wechseln in den Betriebsmodus zumindest einige der folgenden Vorgänge bewirkt: Entdimmen des Displays, Einschalten des Displays und Aktivieren des optischen Herzfrequenzsensors.

15. Am Handgelenk zu tragende Vorrichtung nach Anspruch 14, wobei die am Handgelenk zu tragende Vorrichtung dafür konfiguriert ist, in den Betriebsmodus zu wechseln, wenn die Detektorschaltung eine vorbestimmte Anzahl von aufeinanderfolgenden Nuten erkennt, in denen die Befestigungsklaue im Eingriff ist.

16. Am Handgelenk zu tragende Vorrichtung nach einem der Ansprüche, wobei die am Handgelenk zu tragende Vorrichtung ferner einen Lernmodus umfasst, in dem die Verarbeitungsschaltung dafür konfiguriert ist: das Display zu veranlassen, Anweisungen für den Benutzer anzuzeigen, die durch das Handgelenkband gebildete Schlaufe zu spannen oder zu lockern, und nach jeder angezeigten Anweisung, auf der Grundlage der verarbeiteten Daten im Zusammenhang mit der Herzaktivität, zu ermitteln, ob die Qualität der optischen Herzfrequenzmessung, die von dem optischen Herzaktivitätssensor durchgeführt wird, innerhalb vorbestimmter Werte liegt.

17. Am Handgelenk zu tragende Vorrichtung nach einem der vorstehenden Ansprüche, wobei das Handgelenkband ferner ein Begrenzungselement umfasst, das dafür ausgelegt ist, die durch das Handgelenkband gebildete Schlaufe dahingehend zu begrenzen, dass sie über ein vorbestimmtes Maß festgezogen wird.

## Revendications

1. Appareil porté au poignet (100) pour mesurer une activité cardiaque d'un utilisateur, l'appareil comprenant :
un capteur optique d'activité cardiaque (140) configuré pour mesurer l'activité cardiaque d'un utilisateur par mesure optique de fréquence cardiaque ;
un bracelet (101) adapté pour coupler physiquement le capteur optique d'activité de fréquence cardiaque contre la peau de l'utilisateur en attachant l'appareil porté au poignet autour du poignet de l'utilisateur, le bracelet comprenant un premier (110) et un deuxième (120) élément de connexion courbés, dans lequel le premier élément de connexion est adapté pour glisser à l'intérieur du deuxième élément de connexion, et dans lequel le bracelet comprend au moins une rainure (104) et une griffe de fixation (102) agissant comme contrepartie pour la au moins une rainure, dans lequel
la griffe de fixation est agencée pour mettre en prise la au moins une rainure lorsque le premier élément de connexion est glissé à l'intérieur du deuxième élément de connexion ;
un ensemble de circuits de traitement (610) configuré pour recevoir et traiter des données relatives à l'activité cardiaque provenant du capteur optique d'activité cardiaque ; et un ensemble de circuits de détecteur (620) pour détecter le nombre de rainures mises en prise par la griffe de fixation, et dans lequel l'ensemble de circuits de traitement est en outre configuré pour recevoir des données détectées à partir de l'ensemble de circuits de détecteur et pour calculer le nombre de rainures mises en prise par la griffe de fixation sur la base des données détectées.

2. Appareil porté au poignet de la revendication 1, dans lequel au moins l'un parmi le premier élément de connexion et le deuxième élément de connexion comprend la au moins une rainure.

3. Appareil porté au poignet de l'une des revendications précédentes, dans lequel le bracelet et la griffe flexible sont une entité solidaire.

4. Appareil porté au poignet de l'une des revendications précédentes, dans lequel la au moins une rainure comprend une paroi de fixation, une paroi de serrage et une cavité entre lesdites parois, dans lequel un angle entre une ligne perpendiculaire à l'élément de connexion courbé et la paroi de fixation est plus petit qu'un angle entre ladite ligne et la paroi de serrage, dans lequel la paroi de serrage permet au premier élément de connexion de glisser à l'intérieur du deuxième élément de connexion en serrant la boucle formée par le bracelet en permettant à la griffe de fixation de se dégager automatiquement lorsque la boucle est serrée, et dans lequel la paroi de fixation empêche la griffe de fixation de se dégager de la au moins une rainure dans la direction opposée au serrage.

5. Appareil porté au poignet de l'une des revendications précédentes, dans lequel le bracelet comprend en outre un mécanisme de libération pour permettre à la griffe de fixation de se dégager de la au moins une rainure.

6. Appareil porté au poignet de l'une des revendications précédentes, dans lequel le bracelet comprend une cavité pour le mécanisme de libération.

7. Appareil porté au poignet de l'une des revendications précédentes, dans lequel le mécanisme de libération comprend une surface, et dans lequel la surface est agencée pour s'étendre par-dessus au moins une zone de bord du bracelet.

8. Appareil porté au poignet de l'une des revendications précédentes, dans lequel la griffe de fixation est agencée pour se dégager en appliquant une force mécanique à la surface comprise dans le mécanisme de libération.

9. Appareil porté au poignet de l'une des revendications précédentes, dans lequel le bracelet comprend en outre un indicateur de position indiquant le serrage de la boucle formée par le bracelet.

10. Appareil porté au poignet de la revendication 9, dans lequel l'indicateur de position comprend des indicateurs visuels, dans lequel au moins un des indicateurs visuels est au moins partiellement visible, lorsque le premier élément de connexion est glissé à l'intérieur du deuxième élément de connexion.

11. Appareil porté au poignet de l'une des revendications précédentes, dans lequel l'ensemble de circuits de détecteur comprend un capteur d'accélération.

12. Appareil porté au poignet de l'une des revendications précédentes, comprenant en outre :
un affichage, et dans lequel le nombre de rainures mises en prise par la griffe de fixation est configuré pour être affiché sur l'affichage avec un indicateur visuel.

13. Appareil porté au poignet de la revendication 12, comprenant en outre :
un élément indicateur configuré pour indiquer à l'utilisateur quand le nombre de rainures mises en prise par la griffe de fixation correspond à un nombre prédéterminé.

14. Appareil porté au poignet de l'une des revendications précédentes, dans lequel l'appareil porté au poignet comprend au moins deux modes : un mode veille et mode de fonctionnement, dans lequel l'entrée en mode veille provoque au moins une partie des actions suivantes : l'atténuation de l'affichage, l'arrêt de l'affichage et la désactivation du capteur optique de fréquence cardiaque, et dans lequel l'entrée en mode de fonctionnement provoque au moins une partie des actions suivantes : l'intensification de l'affichage, la mise en marche de l'affichage et l'activation du capteur optique de fréquence cardiaque.

15. Appareil porté au poignet de la revendication 14, dans lequel l'appareil porté au poignet est configuré pour entrer en mode de fonctionnement lorsque l'ensemble de circuits de détecteur détecte un nombre prédéterminé de rainures consécutives mises en prise par la griffe de fixation.

16. Appareil porté au poignet de l'une des revendications, dans lequel l'appareil porté au poignet comprend en outre un mode didacticiel, dans lequel l'ensemble de circuits de traitement est configuré pour : amener l'affichage à afficher des instructions pour que l'utilisateur serre ou desserre la boucle formée par le bracelet et déterminer après chaque instruction affichée, sur la base des données relatives à l'activité cardiaque traitées, si la qualité de la mesure optique de fréquence cardiaque effectuée par le capteur optique d'activité cardiaque se trouve entre des valeurs prédéterminées.

17. Appareil porté au poignet de l'une des revendications précédentes, dans lequel le bracelet comprend en outre un élément limiteur adapté pour limiter le serrage de la boucle formée par le bracelet au-delà d'un niveau prédéterminé.
